# EUROPÄISCHE PATENTSCHRIFT

(11) **EP 0 868 901 B1**
(45) Veröffentlichungstag und Bekanntmachung des Hinweises auf die Patenterteilung: **03.07.2002**
(21) Anmeldenummer: 98102960.6
(22) Anmeldetag: 20.02.1998
(51) Int. Cl.: A61K 7/09

(54) **Verfahren zur dauerhaften Verformung von menschlichen Haaren**
Process for permanent waving of human hair
Procédé pour l'ondulation permanente des cheveux humains

(30) Priorität: 07.03.1997 DE 19709437
(43) Veröffentlichungstag der Anmeldung: 07.10.1998
(73) Patentinhaber: GOLDWELL GmbH, 64297 Darmstadt (DE)
(72) Erfinder: Nöcker, Bernd Dr., 64373 Ober-Ramstadt (DE)

(56) Entgegenhaltungen:
- EP-A- 0 433 663
- EP-A- 0 518 137
- EP-A- 0 604 717
- EP-A- 0 625 350
- EP-A- 0 681 828
- WO-A-88/01161
- WO-A-97/47277
- DE-A- 3 138 813

## Beschreibung

Die vorliegende Erfindung betrifft ein schonendes Verfahren zur dauerhaften Verformung von menschlichen Haaren, das die Eigenschaften des so behandelten Haares gegenüber üblichen Verfahren wesentlich verbessert.

Bekanntlich erfordert die Dauerwellung im Prinzip zwei Behandlungsschritte:

Die reduktive Spaltung der Cystin-Disulfidbrücken des Haares durch Einwirkung eines Reduktionsmittels, und die anschließende Fixierung durch Aufbringung eines Oxidationsmittels, insbesondere Wasserstoffperoxid, wodurch die Cystin-Disulfidbrücken wieder hergestellt werden.

Das klassische Reduktionsmittel ist, wie bereits aus den Pionierpatenten DE-C 948 186 und 972 424 hervorgeht, die Thioglykolsäure, z.B. als Ammonium- oder Monoethanolaminsalz, die in den letzten Jahren durch Glycerinmonothioglykolat teilweise ersetzt wurde; jedoch werden auch Thiomilchsäure und deren Ester sowie, seltener, auch anorganische Sulfite eingesetzt.

Die Thioglykolsäure enthaltenden Zusammensetzungen weisen dabei einen pH-Wert im Bereich zwischen etwa 7,5 und etwa 9,0, insbesondere 8,5 bis 9,0 auf, wobei die Alkalisierung heute in der Regel, neben Ammoniak, durch Zusatz von Ammonium-(bi)-carbonat erzielt wird.

Die wiederholte Einwirkung stark alkalischer Reduktionsmittel und die darauffolgende Fixierung mit Oxidationsmitteln kann insbesondere bei nicht ganz intaktem, d.h. durch Bleichung oder alkalische Oxidationsfärbung vorgeschädigtem Haar, eine weitere Haarschädigung bewirken.

Die vorliegende Erfindung geht daher von der Aufgabenstellung aus, ein Verfahren zur dauerhaften Verformung von menschlichen Haaren zu schaffen, das zu einer schonenden aber wirksamen Verformung des Haares führt, wobei Spannkraft und Elastizität des dauergewellten Haares erhalten bleiben.

Diese Aufgabe wird dadurch gelöst, daß auf das mit Lockenwicklern versehene Haar eine mindestens eine Thioverbindung enthaltende Reduktionsmittel-Zusammensetzung in an sich bekannter Weise zur Einwirkung gebracht, das so behandelte Haar gespült, und dann eine Vorfixierung mit einer Zusammensetzung, die einen Oxidationsmittel-Gehalt von 0,1 bis 1 Gew.-%, berechnet als Wasserstoffperoxid und auf die Gesamtzusammensetzung, aufweist, durchgeführt, und anschließend eine Zusammensetzung mit einem Oxidationsmittel-Gehalt von 1,5 bis 5 Gew.-%, berechnet als Waserstoffperoxid und auf die Gesamtzusammensetzung, zur endgültigen Fixierung auf das Haar aufgebracht wird.

Durch diese "Vorfixierung" mit einer eine niedrige Oxidationsmittel-Konzentration enthaltenden Zusammensetzung wird, für den Fachmann absolut überraschend, eine wesentlich schonendere Haarverformung erzielt, obwohl eher das Gegenteil zu erwarten gewesen wäre.

Vorzugsweise erfolgt die endgültige Fixierung als Schlußbehandlung ebenfalls mit einer relativ niedrigprozentigen Oxidationsmittelzusammensetzung, enthaltend 1,5 bis 2,5 Gew.-% Oxidationsmittel, berechnet als Wasserstoffperoxid.

Gemäß einer bevorzugten Ausführungsform der Erfindung kann die Vorfixierung mit einer üblichen Zwischenbehandlung zwischen Reduzierung und Fixierung verknüpft werden, d.h., einem Zwischenbehandlungsmittel werden 0,25 bis 1 Gew.-% Oxidationsmittel, berechnet als Wasserstoffperoxid, zugesetzt.

Aus der EP 0 433 663 A2 ist ein Verfahren zum Dauerwellen von menschlichen Haaren bekannt, wo die Fixierung mit einer Peroxid-Zusammensetzung die eine Peroxid-Konzentration von maximal 0,3 Gew.-% und einem bevorzugten pH-Wert von 4,7 bis 7,0 in einem Arbeitsgang durchgeführt wird.
Durch dieses Verfahren werden die Vorteile der erfindungsgemäßen Zweistufenfixierung nicht erreicht.

Obwohl Wasserstoffperoxid das für Dauerwellfixierungen meistbenutzte und auch erfindungsgemäß bevorzugte Oxidationsmittel darstellt, können natürlich auch andere Oxidationsmittel, insbesondere Bromate wie Natrium- und Kaliumbromat, Alkaliperoxide wie Natriumperoxid, oder organische Peroxide wie Harnstoffperoxid und Melaminperoxid verwendet werden.

Die erfindungsgemäß in der Reduktionsstufe verwendeten Dauerwellmittel enthalten eine reduzierende Thioverbindung. Bevorzugt sind Thioglykolsäure und Thiomilchsäure sowie deren Salze, insbesondere die Ammonium- und Ethanolaminsalze.

Weitere einsetzbare Thioverbindungen sind insbesondere Cystein bzw. dessen Hydrochlorid, Homocystein, Cysteamin, N-Acetylcystein, Thioglycerin, Ethanolmonothioglykolat, 1,2-Propylenglykolmonoglykolat (vgl. auch WO 93/1791 A1), 1,3-Propandiolmonothioglykolat bzw. das daraus resultierende Isomerengemisch, 1,3-Butandiol- und 1,4-Butandiolmonothioglykolat bzw. deren Isomergemische, Polyethylenglykol- wie Di-, Tri- und Tetraethylenglykolmonothioglykolate, Glycerinmonothiolactate und weitere Thiosäuren und deren Ester sowie Gemische derselben.

Auch die Verwendung anorganischer reduzierender Schwefelverbindungen wie Natriumhydrogensulfit ist prinzipiell möglich.

Der Gesamtgehalt an Reduktionsmitteln in Dauerwellmitteln beträgt üblicherweise 2,5 bis etwa 15 Gew.-%, berechnet auf freie Thioglykolsäure als Bezugssubstanz.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.

Bevorzugte Alkalisierungsmittel sind Ammoniumcarbamat, Ammoniak und/oder Ammonium(bi)carbonat. Es wird die Einstellung eines pH-Wertes im Bereich zwischen etwa 6,5 und etwa 9,5, vorzugsweise etwa 7 bis 8,5, angestrebt.

Dauerwellmittel enthalten vorzugsweise auch Tenside. Deren Anteil liegt bei etwa 0,1 bis etwa 10, insbesondere etwa 1 bis etwa 5 Gew.-% der das Reduktionsmittel enthaltenden Zusammensetzung.

Sowohl bei den in den Reduktionsmittel-Zusammensetzungen als auch bei den in den in den Stufen c) und d) verwendeten Fixiermitteln eingesetzten Tensiden handelt es sich vorzugsweise um die bekannten anionaktiven Produkte, die gegebenenfalls auch in Kombination mit nichtionischen Tensiden z u Einsatz gelangen.

Geeignete anionische Tenside sind insbesondere die bekannten Alkylethersulfate und - carbonsäuren, insbesondere in Form ihrer Alkalisalze, sowie Eiweiß-Fettsäure-Kondensate.

Geeignete nichtionische Tenside sind insbesondere C₈-C₁₈-Fettsäurepolyglykolether, Fettsäurepolyglykolester, Fettsäurealkanolamide, Aminoxide und vor allem C₈-C₁₈-Alkylpolyglucoside.

Es können auch amphotere Tenside wie die bekannten Betaine und Amidobetraine sowie, insbesondere in kationischen Fixierungen, kationaktive Tenside wie quaternäre Ammoniumverbindungen eingesetzt werden.

Ein weiterer wünschenswerter Bestandteil in Redutionsmittel-Zusammensetzungen ist ein C₃-C₆-Alkandiol bzw. dessen Ether, insbesondere Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethylund Monoethylether.

Der Anteil dieser Diole liegt vorzugsweise zwischen 0,5 und 30, vorzugsweise etwa 1 bis etwa 15, insbesondere etwa 5 bis etwa 10 Gew.-% der Reduktionsmittel-Zusammensetzung.

Neben den C₃-C₆-Alkandiolen bzw. deren Ethern können zusätzlich auch Monoalkohole wie Ethanol, Propanol-1, Propanol-2 sowie Polyalkohole wie Glycerin und Hexantriol, Ehtylcarbitol, Benzylalkohol, Benzyloxyethanol sowie Propylencarbonat (4-Methyl-1,3-dioxolan-2-on), N-Alkylpyrrolidone und Harnstoff Verwendung finden.

Weitere mögliche zusätzliche Bestandteile sind kationische, anionische, nichtionische und amphotere Polymere, vorzugsweise in einer Menge von etwa 0,1 bis etwa 5, insbesondere etwa 0,25 bis 2,5 Gew.-% der Gesamtzusammensetzung des Wellmittels bzw. Fixiermittels.

Die im erfindungsgemäßen Verfahren eingesetzten Mittel können selbstverständlich alle in Dauerwellmitteln üblichen Stoffe enthalten, auf deren detaillierte Aufzählung hier verzichtet wird, und als (wäßrige) Lösungen, Emulsionen, Cremes, Schäume etc. vorliegen.

Es kann sich dabei um einphasige Produkte oder um in getrennten Verpackungen untergebrachte Zusammensetzungen handeln, die bei der Anwendung vereinigt werden, wie sie z.B. in der DE 43 04 828 C1 beschrieben sind.

Zur Vermeidung von Wiederholungen wird hierzu auf den Stand der Technik verwiesen, wie er beispielsweise in "Ullmanns's Encyclopedia of Industrial Chemistry", Vol A12 (1986), S. 588 bis 591, sowie insbesondere in der Monographie von K. Schrader, "Grundlagen und Rezepturen der Kosmetika", 2. Auflage (1989, Hüthig-Verlag), S. 823 bis 840, und in dem Übersichtsartikel von D. Hollenberg et. al. in "Seifen-Öle-Fette-Wachse", **117** (1991), S. 81-87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Falls erwünscht, kann vor dem Auftrag des Reduktionsmittels auch noch ein Vorbehandlungsmittel appliziert werden, wie es beispielsweise in der DE 37 40 926 A1 beschrieben ist. Nach dem Aufbringen dieses Vorbehandlungsmittels wird das Haar aufgewickelt und die Reduktionsmittel-Zusammensetzung aufgetragen. Nach etwa 15- bis 30-minütiger Einwirkung und Spülung erfolgt dann die Fixierung auf die erfindungsgemäß definierte Weise.

Die folgenden Vergleichsversuche und die Beispiele illustrieren die Erfindung und deren Effekt.

### Vergleichsversuch

5 Haarsträhnen aus gebleichtem Menschenhaar wurden zunächst mit einer alkalischen Dauerwelle der Zusammensetzung

| | |
|---|---|
| Thioglykolsäure (80%-ig) | 12,5 (g) |
| Ammoniumbicarbonat | 4,5 |
| Ammoniak | pH 8,3 |
| Wasser | ad 100,0 |

20 Minuten bei Raumtemperatur behandelt, danach mit .Wasser gespült, und mit einer Zusammensetzung

| | |
|---|---|
| Wasserstoffperoxid | 0,3 (g) |
| Natriumacetat | 0,5 |
| Essigsäure | pH 4,8 |
| Wasser | ad 100,0 |

5 Minuten vorfixiert, anschließend mit einer Zusammensetzung

| | |
|---|---|
| Wasserstoffperoxid | 2,5 (g) |
| Phosphorsäure | pH 3,5 |
| Wasser | ad 100,0 |

10 Minuten endfixiert, gewaschen und getrocknet.

Weitere 5 Haarsträhnen wurden der gleichen Prozedur unterzogen, allerdings ohne Vorfixierung. Diese Behandlungen wurden an allen Haarsträhnen je dreimal wiederholt. An allen 10 Haarsträhnen wurde der Cysteinsäuregehalt nach den Behandlungen bestimmt.

Bei den nach dem erfindungsgemäßen Verfahren behandelten Haarsträhnen lag der Durchschnittswert bei 1,70 Mol-% Cysteinsäure.

Bei den ohne Vorfixierung behandelten Haarsträhnen lag der Durchschnittswert bei 2,18 Mol-% Cysteinsäure.

Da der Cysteinsäurewert des Haares in direkter Korrelation zur oxidativen Haarschädigung steht, zeigt dieses Ergebnis deutlich, daß durch das erfindungsgemäße Dauerwellverfahren eine signifikant haarschonendere Dauerwellung erzielt wird.

Im folgenden werden weitere Beispiele für im erfindungsgemäßen Verfahren geeignete Vorfixierungs- und Fixierungszusammensetzungen gegeben:

### Beispiel 1

| **Zwischenbehandlungsmittel mit vorfixierender Wirkung** | |
|---|---|
| Wasserstoffperoxid | 0,3 (Gew.-%) |
| Magnesiumsulfat·7 H₂0 | 8,0 |
| Pflanzenproteinhydrolysat | 0,5 |
| Citronensäure·H₂0 | 0,5 |
| Glutaminsäure | 1,0 |
| Glycin | 0,5 |
| Lösungsvermittler | 0,6 |
| Parfum | 0,3 |
| Ammoniak | ad pH 4,5 |
| Wasser | ad 100,0 |

### Beispiel 2

| **Vorfixierung mit konditionierender Wirkung** | |
|---|---|
| Wasserstoffperoxid | 0,5 (Gew.-%) |
| Natriumacetat | 0,5 |
| Kationisches Polymer (Polyquaternium-11) | 0,1 |
| Lösungsvermittler | 0,6 |
| Parfum | 0,3 |
| Essigsäure | ad pH 4,8 |
| Wasser | ad 100,0 |

### Beispiel 3

| **Vorfixierung** | |
|---|---|
| Wasserstoffperoxid | 0,8 (Gew.-%) |
| Citronensäure | 0,5 |
| Ascorbinsäure | 0,1 |
| Cocoamidopropylbetain | 0,2 |
| Ammoniak | ad pH 3,0 |
| Wasser | ad 100,0 |

### Beispiel 4

| **Endfixierung** | |
|---|---|
| Wasserstoffperoxid | 2,0 (Gew.-%) |
| Natriumlaurylethersulfat, 25%-ig | 2,5 |
| Natriumdihydrogenphosphat | 0,2 |
| Parfum | q.s. |
| Phosphorsäure | ad pH 3,0 |
| Wasser | ad 100,0 |

### Beispiel 5

| **Endfixierung** | |
|---|---|
| Wasserstoffperoxid | 1,6 |
| Lauraminoxid | 1,0 |
| Kationisches Polymer (Polyquaternium-2) | 0,3 |
| Natriumdihydrogenphosphat | 0,2 |
| Parfum | q.s. |
| Phosphorsäure | ad pH 4,0 |
| Wasser | ad 100,0 |

### Beispiel 6

| **Zwei-Komponenten-Fixierung** | |
|---|---|
| **Zusammensetzung A** | |
| Wasserstoffperoxid | 5 (g) |
| Phosphorsäure | q.s. |
| Wasser | 10 |

| **Zusammensetzung B** | |
|---|---|
| Cocoamidobetain | 1,0 (g) |
| Cocoamphoacetate | 0,4 |
| 1,2-Propandiol | 2,0 |
| Kationisches Polymer (Polyquaternium-11) | 0,5 |
| Parfum | q.s. |
| Wasser | ad 90 |

Vor der Anwendung werden beide Zusammensetzungen vermischt, wobei eine Fixierlösung mit einem pH-Wert von 3,5 erhalten wird.

Diese Endfixierungen werden im Rahmen des erfindungsgemäßen Verfahrens mit den in den Beispielen 1 bis 3 beschriebenen Vorfixiermitteln eingesetzt.

## Patentansprüche

1. Verfahren zur dauerhaften Verformung von menschlichen Haaren, **gekennzeichnet durch** die Kombination folgender Schritte:
a) Aufbringen und Einwirkung einer mindestens eine Thioverbindung und ein Alkalisierungsmittel enthaltenden Reduktionsmittel-Zusammensetzung auf das Haar;
b) Ausspülen mit Wasser;
c) Aufbringung einer 0,1 bis 1 Gew.-% Oxidationsmittel, berechnet als Wasserstoffperoxid, enthaltenden Zusammensetzung ("Vorfixierung");
d) endgültige Fixierung **durch** Behandlung mit einer 1,5 bis 5 Gew.-% Oxidationsmittel, berechnet als Wasserstoffperoxid, enthaltenden Zusammensetzung; und
e) Ausspülen und Trocknen des Haares.

2. Verfahren nach Anspruch 1, **dadurch gekennzeichnet, daß** die Stufe c) als eine kombinierte Zwischenbehandlung/Vorfixierung durchgeführt wird.

## Claims

1. Process for the permanent waving of human hair, **characterized by** the combination of the following steps:
a) Application of a reducing agent composition comprising at least one thio compound and an alkalizing agent onto the hair;
b) rinsing with water;
c) application of a composition containing 0.1% to 1% by weight of an oxidation agent, calculated as hydrogen peroxide ("pre-neutralizer");
d) final neutralization by treatment with a composition containing 1.5% to 5% by weight of an oxidation agent, calculated as hydrogen peroxide; and
e) rinsing and drying the hair.

2. Process according to claim 1, **characterized by** carrying out step c) as a combination of an intermediate treatment and pre-neutralization.

## Revendications

1. Procédé pour la déformation durable de la chevelure humaine, **caractérisé par** la combinaison des étapes ci-dessous :
a) application et action sur la chevelure d'une composition d'agent réducteur contenant au moins un composé thio et un agent d'alcalinisation
b) rinçage à l'eau ;
c) application d'une composition contenant de 0,1 à 1 % en poids d'un agent d'oxydation, ce pourcentage étant calculé par rapport au peroxyde d'hydrogène ("préfixation") ;
d) fixation finale par traitement avec une composition contenant de 1,5 à 5 % en poids d'un agent d'oxydation, ce pourcentage étant calculé par rapport au peroxyde d'hydrogène ; et
e) rinçage et séchage de la chevelure.

2. Procédé selon la revendication 1, **caractérisé en ce que** l'étape c) est mise en oeuvre comme combinaison du traitement intermédiaire et de la préfixation.
